Europäisches Patentamt

European Patent Office

Office européen des brevets

(11) Publication number: **0 427 997 A2**

# EUROPEAN PATENT APPLICATION

(21) Application number: 90120677.1

(51) Int. Cl.⁵: **A61L 31/00**, A61F 6/04

(22) Date of filing: **29.10.90**

(30) Priority: **10.11.89 IT 2234089**

(43) Date of publication of application:
**22.05.91 Bulletin 91/21**

(84) Designated Contracting States:
**AT BE CH DE DK ES FR GB GR IT LI LU NL SE**

(71) Applicant: **GERMO S.P.A.**
**via Giotto 19/21**
**I-20032 Cormano (MI)(IT)**

(72) Inventor: **Magni, Antonio**
**Via degli Alerami 31**
**I-20148 Milano(IT)**

(74) Representative: **Gervasi, Gemma, Dr. et al**
**NOTARBARTOLO & GERVASI Srl 33, Viale**
**Bianca Maria**
**I-20122 Milano(IT)**

(54) Condom.

A condom is described which is lined inside and outside with a vehicle having a consistency apt to form a thin film, containing a stotal-spectrum disinfectant active against the AIDS virus and against pathogenic agents acting on the sexual organs and which does not irritate the mucous membranes thereof at the concentrations employed.

EP 0 427 997 A2

EP 0 427 997 A2

CONDOM

Starting With the years eighty a previously unknown syndrome has spread, whose morbility rate is in a continuous, very rapid increase. This syndrome, called "acquired immunodeficiency syndrome" or AIDS, from the initials of the English definition, is associated with a profound depression of the cellular immunity. As a consequence, the patient may develop unusual neoplasies and/or opportunistic infections with a very high death rate. The virus which is responsible for the disease was isolated in igais both in France and in the United States and was indicated respectively as LAV and NTLV-III. It is transmitted through certain organic fluids, particularly blood and sperm. The infection may therefore result because of direct contact with such infected secretions, for instance because of punctures or wounds with infected needles or surgical instruments, blood or plasma transfusions or also through sexual intercourse With sick individuals as well as with asymptomatic carriers. AIDS is then an additional illness to those which can be contracted through sexual intercourse and which are for this reason named venereal diseases (syphilis, blennorrhagia, soft sore, venereal lymphogranuloma) making the sexual intercourse greatly more risky in view of the seriousness of the LAV infection. An efficient defense against the risk of contracting AIDS or common venereal diseases in sexual intercourses should be represented by the use of the well known condom. Unfortunately, this preventive means does not present an absolute safety, and a serious margin of risk remains. One should only consider that, according to the literature in the field, the safety presented by the condom, employed as a contraceptive, is not total, the margin of failure being around 10%, either because of lack of experience in the use or because of the presence of micropores in the very thin rubber layer through which seminal fluid may pass, and lastly because of tearing of the condom itself. Obviously, at least the same probability of failure may be expected in the case the condom is employed as a prophylactic in sexual intercourse with subjects at risk. In fact, the sanitary institutions do not consider this means, as produced and sold at present, as absolutely safety means to avoid LAV infections, even if its use may greatly reduce the risk. The same danger exists obviously in the intercourse With carriers of common venereal diseases. The need exists therefore of a prophylactic presenting a maximum of safety against the risk of infections from LAV or other pathogenic agents and which may thus permit of sexual intercourse with subjects with AIDS or seropositive, or with common venereal diseases, with a much greater safety than when a normal condom is used. We now have found that if the condom is covered both inside and outside with a thin layer of a vehicle containing a suitable disinfectant substance which does not irritate the mucous membranes and which is active in general against those pathogenic agents which thrive in the sexual organs (germs, fungi, moulds, viruses, in particular LAV) a means is obtained which answers said needs. It is therefore an object of the present invention to provide a condom covered both inside and outside with a thin layer of a suitable vehicle, which also functions as a lubricant, containing a disinfecting substance which, at the prescribed concentrations, sdoes not irritate the mucous membranes of the sexual organs and which is active against LAV and against the pathogenic agents proper of the environment. As example of such disinfecting agents one can cite phenols, in particular 0-phenylphenol, quaternary salts, in particular benzalkonium chloride, biguanides, in particular chlorhexidine and 2,4-dichlorobenzyl alcohol. Such disinfecting substances are contained in the cream in the following percentages:

| benzalkonium chloride | 0.01 - 3% |
| o-phenylphenol | 0.01 - 2% |
| 2,4-dichlorobenzyl alcohol | 0.01 - 1% |
| chlorhexidine | 0.01 - 2% |

Mixtures of two or more of the above disinfecting substances may be used. The vehicle containing the above disinfecting substances consists of creams, gels or emulsions employed in certain types of condom as lubricants and/or as carriers of substances having particular effects, for instance retarding effect. The compositions according to the invention do not affect the materials of the

2

condom (rubber, caoutchouc, plastic materials) on which they are applied. They are spread on the outside and the inside of the condom With the methods commonly employed for the preparation of lubricated prophylactics or prophylactics containing various principles. The thus prepared condoms are then sealed in envelopes according to known techniques.

## Claims

(57) 1. A condom covered inside and outside with a vehicle of a consistency apt to form a thin film containing at least one total spectrum disinfectant, active against the AIDS virus and against pathogenic agents present in the sexual organs and which, at the concentrations employed, does not irritate the mucous membranes thereof. 2. A condom according to claim 1, characterized in that the disinfectant is selected from the group consisting of benzalkonium chloride, phenols, quaternary salts, biguanides and 2,4-4 dichlorobenzyl alcohol. 3. A condom according to claim 2, characterized in that the disinfectant is benzalkonium chloride at a concentration of between 0.01 and 3%. 4. A condom according to claim 2, characterized in that the disinfectant is 0-phenylphenol at a concentration of between 0.01 and 2%. 5. A condom according to claim 2, characterized in that the disinfectant is 2,4-dichlorobenzyl alcohol at a concentration of between 0.01 and IX. 6. A condom according to claim 2, characterized in that the disinfectant is chlorohexidine at a concentration of between 0.01 and 2%.